(19) Europäisches Patentamt
European Patent Office
Office européen des brevets

(11) **EP 1 696 988 B1**

(12) **EUROPEAN PATENT SPECIFICATION**

(45) Date of publication and mention
of the grant of the patent:
**27.10.2010 Bulletin 2010/43**

(51) Int Cl.:
***A61M 15/00*** *(2006.01)*

(21) Application number: **04804355.8**

(86) International application number:
**PCT/EP2004/014767**

(22) Date of filing: **21.12.2004**

(87) International publication number:
**WO 2005/063322 (14.07.2005 Gazette 2005/28)**

(54) **METHOD FOR PREDICTING DEPOSITION OF INHALED MEDICAMENT AT THE LUNG**

VERFAHREN ZUR VORHERSAGE DER ABSCHEIDUNG VON INHALIERTEM MEDIKAMENT IN DER LUNGE

PROCEDE POUR PREDIRE LE DEPOT D'UN MEDICAMENT INHALE DANS LES POUMONS

(84) Designated Contracting States:
**AT BE BG CH CY CZ DE DK EE ES FI FR GB GR
HU IE IS IT LI LT LU MC NL PL PT RO SE SI SK TR**
Designated Extension States:
**HR LV**

(30) Priority: **23.12.2003 GB 0329884**

(43) Date of publication of application:
**06.09.2006 Bulletin 2006/36**

(73) Proprietor: **GLAXO GROUP LIMITED
Greenford, Middlesex UB6 0NN (GB)**

(72) Inventors:
• **BURNELL, Patricia Kwong Phieu
Ware
Hertfordshire SG12 0DP (GB)**

• **YOUNG, William Kerckhoff
Ware
Hertfordshire SG12 0DP (GB)**

(74) Representative: **Rice, Jason Neale et al
GlaxoSmithKline
Corporate Intellectual Property
980 Great West Road
Brentford, Middlesex
TW8 9GS (GB)**

(56) References cited:
**WO-A-01/74247     WO-A-94/09700**

**Description**

**Technical field**

The present invention relates to a method for predicting the deposition of inhaled medicament in the throat of a patient. The method is particularly useful in predicting the likelihood of throat deposition of medicament, wherein the medicament is arranged for delivery to the patient's lung of a patient by way of an inhaler-type dispenser device.

**Background to the invention**

The use of inhaler devices in the administration of medicaments, for example in bronchodilation therapy is well known. Such devices generally comprise a body or housing within which a medicament carrier is located. Known inhaler devices include those in which the medicament is in dry powder form, including those in which the medicament carrier is a blister strip containing a number of discrete doses of powdered medicament. Such devices usually contain a mechanism of accessing these doses, usually comprising either piercing means or means to peel a lid sheet away from a base sheet. The powdered medicament can then be accessed and inhaled. Other known devices include those in which the medicament is delivered in aerosol form, including the well known metered dose inhaler (MDI) delivery devices. Liquid-based inhaler devices are also known.

Considerable research effort is directed towards the design of new and improved inhaler devices. One important measure of performance of such inhaler devices is the ability to deliver inhaled medicament to the lung of a patient. It is relatively difficult and expensive to conduct performance tests relating to delivery of medicament to the lung performance on live patients (i.e. *in vivo*). A number of standard *in vitro* test methods have therefore been developed in order that inhaler performance may be assessed in the laboratory. Known laboratory test methods included those utilising a pump operated under defined (e.g. standardized) flow conditions and coupled to any of a Marple Miller Impactor (MMI); Twin Impinger (BP); Multi-Stage Liquid Impinger (MLSI); or Andersen Impactor (AI). Other known laboratory test methods utilise apparatus that more or less mimics the action of an inhaling patient. Thus, the inhaler device communicates with an artificial 'mouth' leading respectively to an artificial 'throat', 'respiratory tract' and 'lungs'. The apparatus is arranged such that positive and negative vacuum may be applied in order to simulate the breathing action of a patient. Known apparatus of this sort include the electronic lung.

It is known from both *in vivo* and the above *in vitro* assessments that a significant proportion of medicament inhaled from an inhaler device deposits in the upper respiratory tract, which includes the mouth and throat of a patient, and therefore never reaches its primary therapeutic delivery target point at the lung. Considerable effort has therefore been directed towards understanding pre-lung deposition to enable the design of improved *in vitro* laboratory performance testing apparatus, in particular such apparatus that more effectively simulates what happens *in vivo*.

It has been appreciated that variations in mouth, throat and respiratory tract geometries and dimensions between different patients can affect the tendency for undesirable pre-lung deposition of inhaled medicament. The variation can be particularly broad between paediatric and adult patient groups and even between adults of large build versus smaller adults. For effective simulation of *in vivo* performance, it is therefore desirable to tailor laboratory methods and apparatus for pre-lung throat deposition to take account of the above-described variation in mouth, throat and respiratory tract geometries and dimensions between different patients. Existing methods for measuring patient mouth, throat and respiratory tract geometries, which rely on either assessment of cadavers or on the use of Magnetic Resonance Imaging (MRI) or Computated Tomography (CT) of the throats of live patents, are however expensive and time-consuming and therefore somewhat impractical for commercial use with large patient samples.

The Applicant has now devised a method of assessing pre-lung deposition that both takes account of diverse mouth, throat and respiratory tract geometries and dimensions across patient sample groups and is readily applicable, at reasonable cost, to large patient sample groups. The method relies on the use of acoustic imaging (e.g. acoustic reflection imaging) to map the internal geometry of the mouth, throat and upper respiratory tract of each patient in the sample group. The so-mapped patient geometries are then matched to existing patient geometries derived using current (typically expensive, and time-consuming) methods for which pre-lung deposition data is available or to bent-pipe models to enable pre-lung deposition patterns to be predicted for the acoustically mapped geometries.

The Applicant has also found that pre-lung deposition may be effectively correlated with one or more key internal parameters of a patient's throat. The above-described method may therefore be further simplified by acoustic measurement of these key geometric parameters across the patient sample groups.

PCT Patent Application No. WO 01/74247 describes a method of employing real-time Magnetic Resonance Imaging (MRI) to investigate the effect of patient air way structures on the oral inhalation of a respiratory medicament.

It is an object of the present invention to provide a method for predicting the extent of pre-lung deposition of medicament delivered orally by an inhaler device for patients with a variety of different throat sizes.

It is a further object of the present invention to provide improved laboratory testing apparatus for use in predicting pre-lung deposition of medicament delivered by an inhaler device.

**Summary of the invention**

According to one aspect of the invention there is provided a method for predicting the tendency of inhaled particles to deposit within a first patient's throat when said particles are inhaled through an airway defined by said first patient's throat, said method comprising
determining at least one internal physical parameter of said airway defined by the first patient's throat by means of acoustic imaging of the airway defined by the first patient's throat; and
matching said at least one internal physical parameter of the airway of the first patient's throat with a dataset comprising pre-determined data relating to the corresponding internal physical parameter for the throat of at least one other patient, wherein said dataset also comprises pre-determined data relating to the tendency of said inhaled particles to deposit within said at least one other patient's throat, and said matching thereby enables prediction of the tendency for the inhaled particles to deposit within the first patient's throat.

There is provided a method for predicting the tendency of inhaled particles to deposit within a first patient's throat. That is to say, the method enables prediction of the tendency of particles to deposit within a first patient's throat when said particles are orally inhaled through said first throat.

The method is suitable for the predictive assessment of particle deposition within a patient's throat to which particulate product is delivered by a delivery system (e.g. from an inhaler device). In general terms, the method is suitable for use in predictive assessments where both throat internal physical parameter data and throat particle deposition data exists for at least one other patient (e.g. in an existing patient database).

The method also enables prediction of lung deposition of the particles, which may be obtained by subtracting the number, mass (or %) of particles deposited on the throat from the total number of particles (i.e. initially 100%) inhaled by the patient.

Suitable particles typically comprise particles of medicament either in the form of a formulated medicated product or as pure drug or alternatively, the particles may comprise placebo. Suitably, the particles are deliverable by means of an inhaler-type delivery device (e.g. a dry powder inhaler (DPI) device for the delivery of dry powdered medicament or medicament formulation; or metered dose inhaler (MDI) device for the delivery of aerosol medicament formulation).

In the method, at least one internal physical parameter of the airway defined by the throat of the first patient is determined by means of acoustic imaging (e.g. acoustic reflection imaging) of the airway defined by that first patient's throat.

The term throat herein is essentially used to mean that part of the human body (or suitable laboratory model thereof) encountered by particles delivered to a mouth for inhaled transport to the lung that occurs prior to the lung. As used herein, the term throat therefore encompasses the mouth cavity, pharynx, epiglottis, larynx and trachea and any defined separate part thereof. In one aspect, the throat is taken to comprise that part of the mouth cavity and respiratory tract down to the patient's fifth vertebrae.

The term throat airway herein is used to mean that airway (or airpath) defined by the inner walls of the throat. It will be appreciated that throat deposition potentially occurs on said throat walls and also on any structures within the airway when particles are drawn through the airway defined thereby.

Suitable physical parameters that may be used to define the airway defined by the throat include the throat volume, area of a suitable cross-section (e.g. that taken in a coronal plane) and length of the throat (e.g. taken in the central sagittal plane).

The at least one internal physical parameter of the airway defined by the throat of the first patient is determined by acoustic imaging thereof such as by an acoustic imaging method using acoustic reflection. A suitable method of acoustic reflection imaging involves the use of an acoustic pharyngometry apparatus. One such suitable apparatus is sold by Hood Laboratories of 575 Washington St, Pembroke, MA 02359, United States of America under the trade name Eccovision and described in the Operating Manual therefor.

Suitable apparatus for acoustic pharyngometry are further described in PCT Patent Application No. WO 94/09700 also in the name of Hood Laboratories and comprise a hand-held acoustic imaging head which is rugged and entirely hand supportable and operable by an operator, throughout an imaging procedure, which head comprises;

A. a rugged hand-holdable housing having

1. an elongate body, defined by

(a) a top end;
(b) a base end;

(c) an outer wall extending between the top end and the base end; and
(d) an internal chamber;

2. an aperture through the housing top end, providing fluid communication between the internal chamber and the outside of the housing; and
3. a shape and configuration of the outer wall facilitating gripping of the housing with a human hand;

B. an acoustic pipe for transmitting acoustic energy and receiving the reflected acoustical energy, mounted in the aperture, said pipe having a first end within the chamber and an open second end outside of the housing, said second end of the acoustic pipe being adapted for connection of the acoustic pipe to an orifice leading into the respiratory tract;

C. a launching transducer mounted in the chamber and coupled to the first end of the acoustic tube, for launching acoustical energy into the acoustic pipe, propagating an incident wave out of the open second end;

D. at least one acoustic pressure wave sensing transducer mounted on the acoustic pipe at a location between the first and second ends of the acoustic pipe, for sensing reflections of the incident wave, received back in the acoustic tube through the open second end and generating a signal; and

E. means at least partially within the chamber, connected to the acoustic wave sensing transducer, for transmission of signals transduced, to processor means for processing said signals into a processor output signal characteristic of the morphology of a site within the respiratory tract of the patient.

Acoustic reflection methods and apparatus have also been described in the following journal articles:

J Appl Physiol. 1984 Sep;57(3):777-87. Reproducibility and accuracy of airway area by acoustic reflection. Brooks LJ, Castile RG, Glass GM, Griscom NT, Wohl ME, Fredberg JJ.

Am Rev Respir Dis. 1987 Feb;135(2):392-5. Airway area by acoustic response measurements and computerized tomography. D'Urzo AD, Lawson VG, Vassal KP, Rebuck AS, Slutsky AS, Hoffstein V.

Eur Respir J. 1991 May;4(5):602-11. The acoustic reflection technique for non-invasive assessment of upper airway area. Hoffstein V, Fredberg JJ.

Ann Biomed Eng. 1995 Jan-Feb;23(1):85-94. Measurement of upper airway movement by acoustic reflection. Zhou Y, Daubenspeck JA.

J Appl Physiol. 1994 May;76(5):2234-40. Pulmonary airway area by the two-microphone acoustic reflection method. Louis B, Glass GM, Fredberg JJ.

Physiol Meas. 1993 May;14(2):157-69. Acoustic reflectometry for airway measurements in man: implementation and validation. Marshall I, Maran NJ, Martin S, Jan MA, Rimmington JE, Best JJ, Drummond GB, Douglas NJ.

J Appl Physiol 2000 Apr;88(4):1457-66. A new nasal acoustic reflection technique to estimate pharyngeal cross-sectional area during sleep. Huang J, Ital N, Hoshiba T, Fukanaga T, Yamanouchi K, Toga H, Takahashi K, Ohya N.

The method provided herein involves matching said at least one internal physical parameter of the airway defined by the first patient's throat and determined by acoustic imaging of that airway with a dataset comprising pre-determined data relating to the corresponding internal physical parameter for the (airways defined by the) throat of at least one other patient, typically the throats of each of plural other patients. That is to say, in a pre-step, data relating to the corresponding internal physical parameter is collected for the throat of at least one other patient, typically for each of plural other patients, and that data collated as a dataset, to which comparison for matching purposes with the at least one internal physical parameter of the first patient's throat may be made.
The internal physical parameter data of the pre-determined dataset may be collected from the airway defined by the throat of each of the at least one other patient by any suitable method.
In one embodiment, the pre-determined dataset comprises internal physical parameter data for the throats of plural other patients, for example at least ten, preferably at least twenty, more preferably as large a sample as possible of other patients.

In another embodiment, the data is collected by study of just one other patient by varying the particle size of the particles inhaled and/or the inspiratory flow profile for that patient's throat.

In one embodiment, data is collected by use of Magnetic Resonance Imaging (MRI) of the throat airways particular to the plural other patients. Such MRI techniques are known to be expensive and time-consuming, so for commercial reasons it may be necessary to limit the dataset to a relatively small number of patients, but not so small that the quality (i.e. for matching purposes) of the dataset is compromised.

References describing MRI imaging of patient throats include:

3D reconstruction of the upper airway during inhalation from drug delivery system using MRI. Ehtezazi T, Horsfield MA, Barry P and O Callaghan CO, (2000) Proceedings of Drug Delivery to the lungs XI, pages 90-93;

Lung Air spaces: MR Imaging evaluation with hyperpolarized 3He gas, de Lange EE, Mugler JP III, Brookeman JR, Knight-Scott J, Truwit JD, Teates CD, Daniel TM, Bogorad PL, Cates GD. Radiology, 1999, 210 (3), pages 851-857;

McRobbie DW, Pritchard S, Quest R (2003) Studies of the human oropharyngeal airspaces using magnetic resonance imaging I. Validation of a three-dimensional MRI method for producing ex vivo virtual and physical casts of the oropharyngeal airways during inspiration. J Aerosol Medicine 16: 399-413;

McRobbie DW, Pritchard SE, Quest RA. Volumetric studies of the oropharyngeal airways by 3D MRI. International Society for Aerosols in Medicine, 13th International Congress; 17-21 Sep 2001; and

McRobbie D.W., Quest R.A., Pritchard S. (2000) "Pulse Sequences for Respiratory Gated MR Virtual Bronchoscopy", International Society for Megnetic Resonance in Medicine, 8h ann. Mtg, Denver no. 1749.

In another embodiment, the data is collected by use of bent-pipes simulating various throat models with suitably selected patient throat dimensions.

The pre-determined dataset suitably also comprises data relating to the particle size distribution of the particulate product to be inhaled. Aerosol compositions, for example, typically have a particular particle size distribution.

The pre-determined dataset also comprises data relating to the tendency of said inhaled particles to deposit within each of said at least one other patient's throats. That is to say, for each patient the dataset comprises at least a first data point relating to the at least one internal physical parameter relevant to that patient's throat airway and at least a second data-point relating to the tendency of inhaled particles to deposit within that patient's throats on inhalation of particles through that patient's throat.

Patient throat deposition data is typically obtained by making a model reconstruction of the patient's throat based upon dimensional and geometric information obtained by previous measurements. A particulate sample product of known composition and physical characterisation is then caused to be inhaled through the reconstructed throat. Deposition of particulate material in the throat is assessed using a method such as gamma scintigraphy or gravimetry or other methods known in the art.

In the method, the at least one internal physical parameter of the first patient's throat airway is matched with the dataset and that matching enables prediction of the tendency for the inhaled particles to deposit within the first patient's throat.

Any suitable matching process is envisaged including those relying on statistical methods such as curve-fitting methods, as are known in the art.

In an embodiment of the invention the dataset comprises data relevant to each of plural patients and is assembled by

(i) determining at least one internal physical parameter of the airway defined by the throat of each other patient; and
(ii) determining the tendency of inhaled particles to deposit within the throat of each other patient.

The step of assembling a dataset comprising physical parameter and particle deposition data relevant to the at least one other patient essentially comprises a pre-step whereby a reference dataset is compiled for use in the later matching with the at least one internal physical parameter of the airway defined by the first throat to enable the first throat's tendency for inhaled particle deposition to be predicted.

In one embodiment, the dataset comprising physical parameter and particle deposition data relevant to the at least one other patient is obtained by use of a laboratory research method for predicting the tendency of particles to deposit within a throat when said particles are inhaled through an airway defined by said throat, and in which the method comprises

measuring the volume (V) of said throat airway;

measuring the path length (L) of a central line of the throat airway in the mid-sagittal plane;
measuring the flow rate (Q) of said particles or the airflow in which said particles are suspended;
calculating a mean throat diameter ($D_{mean}$) by means of the formula

$$D_{mean} = 2 (V / \pi L)^{0.5} \qquad\qquad (1)$$

calculating a mean particle flow velocity ($U_{mean}$) by means of the formula;

$$U_{mean} = QL / V \qquad\qquad (2)$$

and predicting the amount of particle deposition (P) at the throat by correlating terms defined by the formula

$$P = f(U_{mean} / D_{mean}) \qquad\qquad (3)$$

wherein P is a function of $U_{mean}$ and $D_{mean}$.

In one embodiment, the amount of particle deposition (P) at the throat by is correlated with a Stokes number (StK) defined by the formula

$$Stk = Z (U_{mean} / D_{mean}) \qquad\qquad (3A)$$

wherein Z is a parameter characteristic only of said particles and said air and having units such that Stk is a dimensionless number.

A laboratory research method is described in detail in the following article: In Vitro Intersubject and Intrasubject Deposition Measurements in Realistic Mouth-Throat Geometries; B Grgic, W H Finlay, P K P Burnell, AF Heenan, Aerosol Science 35 (2004) 1025-1040.

It will be appreciated that, in accord with this method of providing the dataset, for a fixed particle sample and fixed air condition the amount of particle deposition will correlate only with the values of $U_{mean}$ and $D_{mean}$ both of which are readily determinable. The method therefore allows for effective and straightforward predictive modelling of throat deposition.

The method predicts the amount of deposition of particles drawn through an airway defined by a throat by inhalation (e.g. by an orally inhaled air flow). The method (with suitable modification) may be used for the predictive assessment of particle deposition within a throat to which particulate product is delivered by an inhaler device, and hence for predicting effectiveness of deposition to the lung. The method may also be used in predictive assessments by reference to existing throat deposition profiles for known throat geometries.

In the method for providing the dataset, readily measurable parameters are used to characterise the throat. The first parameter is the volume (V) of the airway defined by the throat, which may be for example be measured by Magnetic Resonance Imaging (MRI) of the throat of the patient as described in PCT Patent Application No. WO 01/74247. Typical human throat airway volumes (V) are of the order of from 25 to 100 cm$^3$.

The second parameter is the path length (L) of a central line of the airway defined by the throat measured in the mid-sagittal plane. Typical human adult throat path lengths (L) are of the order of from 15 to 24 cm but will be less for small adults and/or children.

The method also involves calculating a mean throat diameter ($D_{mean}$) by means of the formula:

$$D_{mean} = 2 \, (V / \pi L)^{0.5} \qquad\qquad (1)$$

A readily measurable parameter is also used to measure the flow rate (Q) of the particles in the air flow that is created by inhalation through the throat. In this method, particle flowrate may be taken to be equal to the airflow rate because of their close association. The peak flow rate for inhalation through a human throat typically has values of from 30 to 165 litres/minute (e.g. when a patient inhales orally through a dry powder inhaler).

The method then involves calculating a mean flow velocity ($U_{mean}$) within the air flow in the throat by means of the formula:

$$U_{mean} = QL / V \qquad\qquad (2)$$

The particle flow rate (Q) is conveniently measured by use of a calibrated flow meter. Typical flow rates are from 10 to 100, particularly from 30 to 90 litres / second.

The amount of particle deposition (P) at the throat is correlated with the inertial parameter, the Stokes number (StK) defined by the formula

$$Stk = Z \, (U_{mean} / D_{mean}) \qquad\qquad (3)$$

wherein Z is a parameter characteristic only of the particles and the air and having units such that Stk is a dimensionless number.

Thus, for a selected particle sample and air conditions Z is a constant and the amount of deposition correlates with $U_{mean}$ and $D_{mean}$. Where $U_{mean}$ is also constant the deposition correlates with $D_{mean}$, which value is readily obtainable by measurement of a patient's throat.

In more detail, the amount of particle deposition (P) at the throat is predictable by correlation with Stk defined by the formula:

$$Stk = \rho \, d_p^2 U_{mean} / 18\mu D_{mean} \qquad\qquad (4)$$

wherein $\rho$ is the density of the air (usually measured in kgm$^{-3}$); $\mu$ is the fluid dynamic viscosity (usually measured in kgm$^{-1}$s$^{-1}$); and $d_p$ is the mean diameter of the particles (usually measured in m).

Thus, Z may also be expressed as:

$$Z = \rho \, d_p^2 / 18\mu \qquad\qquad (5)$$

The correlation of amount of particle deposition (P) at the throat with the Stokes number (StK) is particularly appropriate where impaction is the primary reason for particle deposition. Impaction tends to occur where a high-speed fluid flow of particles in the airway defined by the patient's throat undergoes a change in flow direction (i.e. inertial impaction governs).

For certain throat geometries, changes in air/particle flow profile associated with rapid changes in throat geometry experienced by the flowing particles can also lead to throat deposition.

To take account for changes in air/particle flow profile it is useful to define a Reynolds number ($R_e$) correction wherein

$$R_e = \rho\, U_{mean}\, D_{mean}\, /\, \mu \qquad\qquad (6)$$

Formula (6) may also be expressed as:

$$R_e = (2\rho Q\, /\, \mu)(L\, /\, V)^{0.5} \qquad\qquad (6a)$$

The Reynolds number, a measure of the level of turbulence in the system, is employed to provide an empirical correction wherein the Stokes number (Stk) is multiplied by the Reynolds number ($R_e$) to the power of 0.37 and thus to predict the amount of particle deposition (P) at the throat by correlating with the expression Stk. $R_e^{0.37}$.

A plot of P versus Stk. $R_e^{0.37}$ for different particle inhalation profiles at the throat has been found to give a good correlation, which may be exploited in straightforwardly predicting the amount of particle deposition values for given values of $U_{mean}$ and $D_{mean}$. The correlation often takes the form of a sigmoidal (i.e. S-curve) relationship that is straightforwardly mapped using known curve fitting methods.

The Applicant has found that the predictive method described herein is particularly suitable for assessment of throat deposition where the medicament to be inhaled is supplied from a passive delivery system, in which the principal (or indeed, total) source of energy to draw the medicament into the lung is supplied by the patient's breath. Passive delivery tends to be employed in dry powder delivery devices wherein the patient's breath is harnessed to aerosolise the dry powder dose. Passive delivery is also typically the mechanism in nebuliser type delivery systems, in which the medicament to be inhaled in supplied as a relatively passive cloud. Both of these passive systems contrast with the well-known metered dose inhaler ('puffer') type device in which an energised puff of aerosol cloud is supplied for inhalation.

The predictive method described herein is also suitable for assessment of throat deposition where the medicament to be inhaled is supplied from a more active delivery system, in which the inhaler provides initial energy (e.g. kinetic energy) to the medicament to be inhaled (e.g. release of aerosolised medicament from a metered dose inhaler). Further pre-steps may be involved when the methods are so-employed such as the creation of a dataset specific to the particular active delivery system (e.g. particular to MDIs).

It will be appreciated that steps of the method herein, particularly data processing and matching steps, are susceptible to being carried out by a suitably programmed computer.

At least one step of the method described above may be implemented in the form of computer software. The software may comprise a computer program comprising program code means for, when executed on a computer, instructing a computer to perform some or all of the steps of the method. The software may also comprise a computer program product comprising a computer readable recording medium having recorded thereon a computer program comprising code means for, when executed on a computer, Instructing said computer to perform some or all of the steps of the method.

The invention will now be described with reference to the accompanying drawings in which:

Figure 1 shows a cross-sectional view of a patient's head and upper respiratory tract taken along the sagittal plane;

Figure 2a shows a cross-sectional view of the airflow on inhalation through an airway defined by a patient's throat taken along the sagittal plane;

Figures 2b and 2c shows cross-sectional views of particle deposition on inhalation of particles of respective sizes $3\mu$m and $5\mu$m through an airway defined by a patient's throat taken along the sagittal plane;

Figure 3 shows a flow diagram of the steps involved in creating a reference database comprising MRI-derived throat physical parameter data and throat deposition data from a patient sample;

Figure 4 shows a schematic representation of a suitable experimental set-up to measure throat deposition data using a model throat;

Figure 5 shows a plot of deposition efficiency (%) versus inertial parameter for data gathered using different patient throat models;

Figure 6 shows a plot of deposition efficiency (%) versus Stokes number (Stk) calculated using $U_{inlet}$ for data gathered using the different patient throat models of Figure 5;

Figure 7 shows a plot of deposition efficiency (%) versus Stokes number (Stk) calculated using $U_{mean}$ for for data gathered using the different subject throat models of Figure 5;

Figure 8 shows a plot of deposition efficiency (%) versus corrected Stokes number (Stk) calculated using $U_{mean}$ for for data gathered using the different subject throat models of Figure 5;

Figure 9 shows a plot of dose (% label) versus throat volume ($cm^3$) for data gathered using different subject throat models;

Figure 10 shows a plot of dose (%) versus velocity within throat ($cms^{-1}$) for data gathered using different subject throat models;

Figure 11 shows a flow diagram of the steps involved in creating a patient dataset comprising acoustic imaging-derived throat physical parameter data and throat deposition data predicted by matching with a reference database; and

Figure 12 shows a perspective view of a set up for the measurement of patient throat data using an acoustic pharyngometry apparatus.

**Detailed Description of the Drawings**

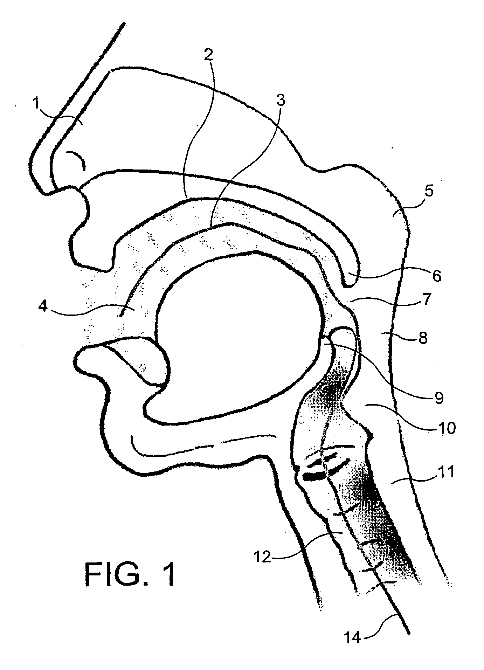

Figure 1 shows a cross-sectional view of a patient's head and upper respiratory tract taken along the sagittal plane, that is to say the plane that defines the left hand side of the patient's body from the right hand side thereof.

In more detail, Figure 1 respectively shows the nasal cavity 1, hard palate 2, oral cavity 3, tongue 4, nasopharynx 5, soft palate 6, uvula 7, pharynx 8, epiglottis 9, laryngo-pharynx 10, oesophagus 11 and laryngal cavity 12 of the patient. Also shown is central line 14 of the throat airway of the patient in the sagittal plane. In accord with the method herein, the path length (L) of the throat of the patient is measured along the central line 14.

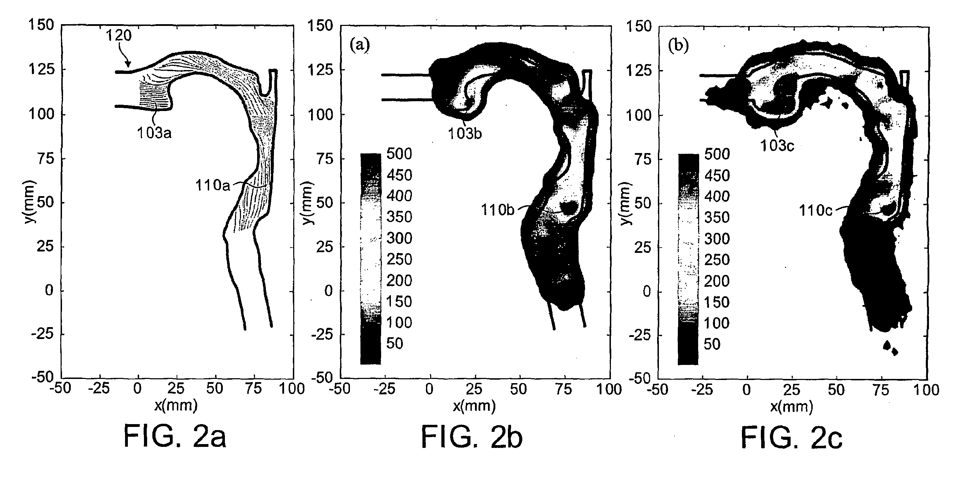

Figure 2a to 2c shows corresponding cross-sectional views of an airway defined by a patient's throat 120 taken along the sagittal plane.

Figure 2a shows the flow pattern created when the patient inhales through the airway defined by the throat at a typical flow rate of 90 litres per minute. It may be seen that the pattern of air flow within the patient's throat is non-uniform with regions of high change in flow velocity at the back of mouth 103a and at the epiglottis 110a shown by closely spaced lines of flow.

Figures 2b and 2c show an illustrative particle deposition profile created when the patient inhales particles through the airway defined by the throat at a typical flow rate of 90 litres per minute. In Figure 2b the particles have a mean particle size of $3\mu m$ and in Figure 2c the particles have a mean particle size of $5\mu m$. It may be seen that the particle deposition profile within the patient's throat is also non-uniform with regions of high deposition observed at the back of the mouth 103b, 103c and at the epiglottis 110b, 110c shown by dark regions on each respective Figure.

Reference dataset compilation

1. First reference database

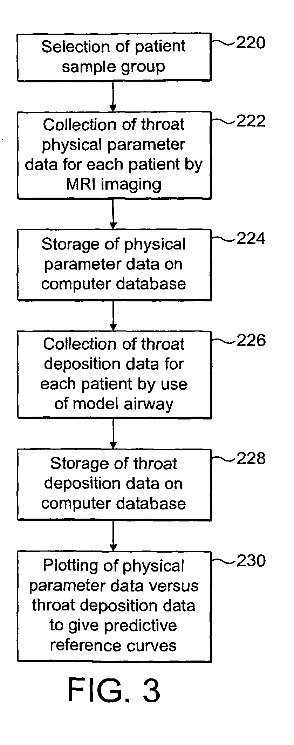

In accord with the method herein, a first reference dataset comprising throat physical parameter data was collected from the throats of an initial sample of twenty subjects. The steps involved in assembling this first reference dataset may be better understood by reference to the flow diagram of Figure 3.

Initially, a sample group was selected 220 to comprise subjects of different builds (i.e. large and small adults) to give a good range of throat sizes. Physical parameter data was then collected 222 for each patient by use of Magnetic Resonance Imaging (MRI) of the throat airway of each patient in the sample group. MRI Imaging is a known technique for collection of such data. The physical parameter data collected for each patient included amongst others: the volume of the airway defined by the patient throat; the cross-section area of that airway measured in various planes including the sagittal, coronal and axial; and length of that airway also measured in the central sagittal plane. The physical parameter data for each patient was stored as a dataset on a computerized database 224.

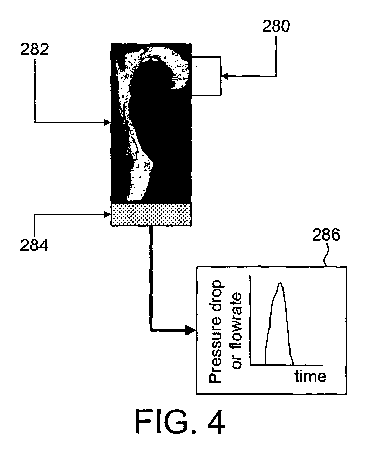

Throat deposition data was also collected for the each patient 226 of the same initial patient sample by requiring a defined particle sample to be inhaled through a model airway defined by and constructed according to the previously collected physical throat parameters of each patient under measurable inhalation flow conditions. A schematic representation of a suitable experimental set up is shown at Figure 4, in which inhaler 280 is arranged to provide aerosolised medicament dose for inhalation through the airway defined by throat model 282. The throat model 282 comprises a three-dimensional model (typically formed of moulded plastic) of the patient throat and airway. Filter 284 is employed to collect the ex-throat medicament dose. Inhalation profile recorder 286 is used to both measure and graphically represent the inhalation profile (e.g. pressure drop or flow rate vs. time) through the throat model 282. The throat deposition data for each patient throat is stored as a corresponding dataset on the computerized database 228.

A first reference computerized database was thus, assembled to comprise throat physical parameter and corresponding throat deposition data for each patient in the sample group. Using appropriate plots, the throat deposition data was plotted against the throat physical parameter data to give reference curves 230 that might be used in a predictive sense (e.g. to predict throat deposition for a new patient for whom suitable throat physical parameter data is known).

In more detail, deposition efficiency, which measures the percentage of particles deposited in the throat, was plotted against various mathematical expressions defined by physical parameters characteristic of the particles, air flow and throat geometry to establish any correlations. Eight throat models were employed and these were designated AA, AB, AC, AD, AE, AF, AG and AH. All were obtained by reference to MRI scans taken of subject throat geometries.

In more detail, in order to obtain physiologically realistic mouth and throat geometries, accurate anatomical models were obtained using the well-established non-invasive medical imaging technique of magnetic resonance imaging (MRI). The subjects were in a supine position and data were acquired upon inspiration. A low resistance pressure monitoring device included as a part of mouthpiece was used to monitor pressure changes and trigger scanning at 25% of maximum inspiratory pressure drop in the mouthpiece. The total mouth and throat geometry is made up of 120 scans. Spatial resolution is 1 x 1 x 1.25mm. The image volume files were converted into 3D volume files and then to the STL file format. CAD designs of models based on the STL geometries were developed using DeskArtes 3Data Expert (DeskArtes, Helsinki, Finland). The models were made as shells with a uniform thickness of 6 mm. Inlet and outlet orifices in the form of straight tubes, with inlet diameter being the same as the mouthpiece diameter used during the *in vivo* scans, were included in the design at this stage. Manufacturing of the cases was done using a FDM 8000 rapid prototyper (Stratasys. Eden Prairie.MN), which uses a fused deposition modelling process and process solid copies of the 3D CAD designs in acrylonitrile-butadiene-styrene (ABS) plastic. The resolution of the manufactured model is 0.127mm. The ABS plastic models are relatively durable and can withstand subsequent testing, drilling and painting.

A set of seven mouth and throat realistic models was obtained as described above. These models were a subset chosen to represent the full range of the key mouth-throat dimensions in the larger set of 80 patient throat geometries (derived from 20 patients) obtained from MRI scans using a convex hull statistical to ensure this subset covers the multi-dimensional geometric space associated with these dimensions in the larger set. Thus, the seven models can be considered as being the most complete possible such subset. The three models designated as AB, AE and AA are from three different individuals. The models designated as AG and AH, and AC and AD are two sets of intrasubject geometric configurations. One additional model designated as AF, included in the results and discussion section, was built up of regular geometric shapes and represents an average geometry of actual subjects. For a full description of the design of the AF geometry see Stapleton KW, Guentsch E, Hoskinson M.K. and Finlay WH (2000): On the Suitability of K-E Turbulence Modelling for Aerosol Dispersion on the Mouth and Throat: A Comparison with Experiment, J. Aerosol Sci, 31, 739-749.

Monodisperse particles of di-2-ethyhexyl sebacate oil (DEHS) were generated using a controlled heterogeneous condensation aerosol generator (CMAG, Model 3475. Topas, Germany). Particle sizes and monodispersity were monitored using a Mach II Aerosizer (TSI, St. Paul, MN). Particles were radiolabelled with technetium $^{99m}$Tc. The flow rate was regulated and measured with a calibrated rotameter (Omega, Stamford, CT), which was corrected for the actual pressure level in the system. The outlet from the mouth and throat cast was attached directly to Marquest respirguard filters (#303, Marquest, Boulder, CO) and then to a vacuum pump (GAST MFG Corp., Brenton Harbor, MI) via the flowmeter. After completion of the test, the models were disassembled along their mid-sagittal planes into halves.and together with the filters imaged by a single photon emission gamma camera (Marconi/Prism Axis 2000 Picker, Cleveland, OH) using a low energy high resolution (LEHR) collimator. The resulting images were superimposed with separately drawn contours of regions of interest. Planar projections of the models' physical boundaries and corresponding subregions were obtained from planar images of the models' filled with radioactively-laced water. Image analysis was done using customized image analysis software MEDisplay 98 (MEDisplay Systems Inc., Edmonton, AB).

The mouth and throat model was divided into 4 subregions: 1) the oral cavity or mouth (also called the buccal cavity) which extends from the back of the teeth to the uvula; 2) the nasopharynx-epiglottis from the nasopharynx to the tip of epiglottis; 3) the larynx from the tip of the epiglottis to just below the vocal cords and 4) the trachea. Deposition in each region was determined as a fraction of total particles entering the mouth. Each test was repeated three times, except for the regional deposition data for the AA, AC and AD models, which were obtained using single experiments. Subsequently, the mean and standard deviation were determined for each experimental condition.

In order to allow more rapid data acquisition, a gravimetric method was also used for the AA, AC and AD models to measure total particle deposition, using the same experimental setup. The cast and filters were simply weighed before and after particle collection, and collected mass was determined. The two methodologies were cross-checked and good agreement was found.

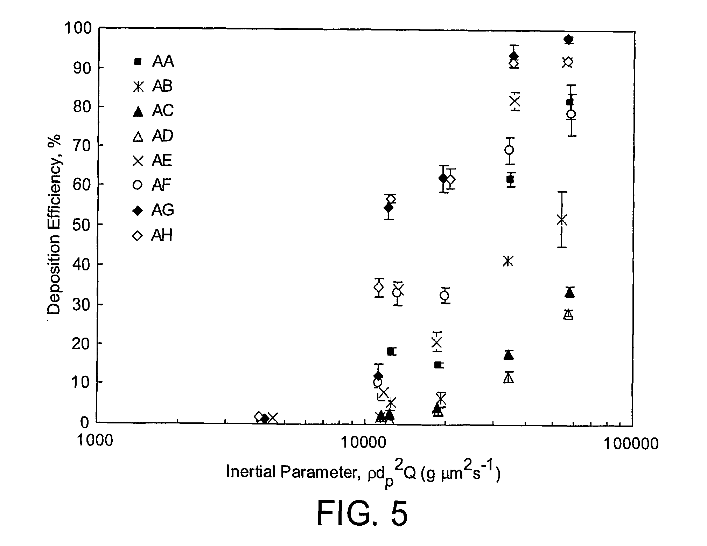

Figures 5 to 8 show plots of particle deposition efficiency (% of particles deposited within throat) versus different mathematical expressions based on data gathered using different subject throat models.

In more detail, Figure 5 shows a plot of the inertial parameter $d_p^2Q$, where dp is the particle diameter in $\mu$m and Q is the flow rate in $cm^3/s$, against deposition efficiency. The scatter in data is in part, due to intersubject variations and different inlet diameter conditions. Intrasubject variability (AG vs. AH or AC vs. AD) is seen to be considerably smaller than intersubject variability.

While the inertial parameter is the traditional independent variable used when presenting mouth and throat deposition, it does not account for the different geometry and inlet diameter conditions. Instead a Stokes number is used, which takes into account the relevant length and velocity scales. The Stokes number is calculated as:

$$Stk = \rho\, d_p^2 U\, /\, 18\mu D \qquad\qquad (4b)$$

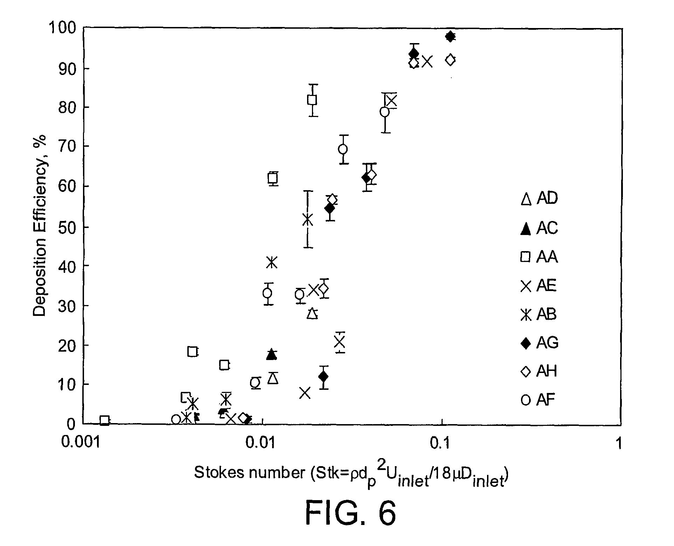

Where, U is a velocity scale (m/s), $\mu$ is the fluid dynamic viscosity (kg/m s) and D is a corresponding length scale (m) for the specific geometry or inlet conditions. Inlet diameter is believed to have an effect on mouth and throat deposition especially for mouth deposition and appears to be a reasonable choice for a length scale D. Using inlet diameter and inlet mean velocity to calculate Stokes number, particle deposition efficiency vs. Stokes number is shown in Figure 6. Plotting the particle deposition efficiency vs. inlet Stokes (i.e. calculated using $U = U_{inlet}$, the flow value at the inlet to the throat model) number collapses the data much better than the inertial parameter, but there is still significant scattering due to the different geometric configuration downstream of the inlet.

In a development of the approach the geometry specific D in equation (4b) is calculated as a mean or equivalent throat diameter $D_{mean}$ calculated simply by dividing cast volume V by the path length L of the central sagittal line of the model to give an area. Assuming a circular equivalent (mean) cross-sectional area of the throat, an equivalent diameter is derived as:

$$D_{mean} = 2\,(V\, /\, \pi L)^{0.5} \qquad\qquad (1)$$

A corresponding mean flow velocity ($U_{mean}$) is calculated from the volume flow rate and the mean cross-sectional area:

$$U_{mean} = QL\, /\, V \qquad\qquad (2)$$

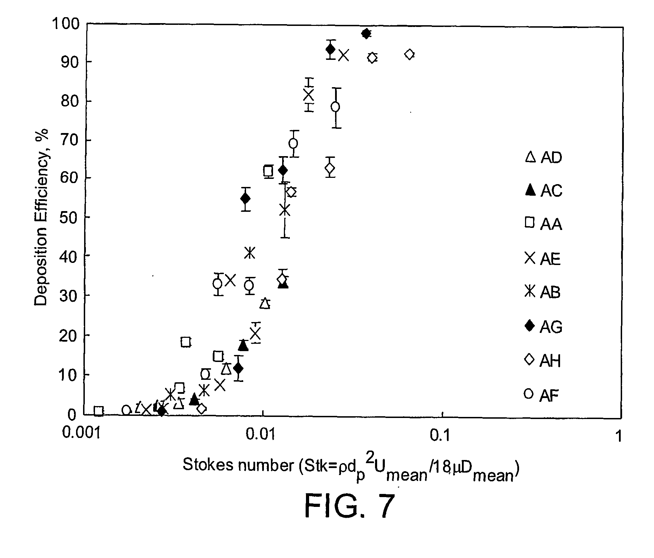

The resulting plot is shown in Figure 7. When the data are plotted using the equivalent diameter $D_{mean}$ and velocity $U_{mean}$ in the Stokes number, the scatter among the data is markedly reduced (compare Figure 7 to Figure 6). That is to say, the correlation between deposition efficiency and the Stokes number calculated using $D_{mean}$ and $U_{mean}$ values is much better, in accord with the present invention. Previous deposition tests suggested a possible Reynolds number effect on deposition since data taken at each flow rate followed a distinct curve on the Stokes number vs. deposition efficiency plot. Deposition experiments in the AF geometry with constant Stokes number and varying Reynolds number demonstrated that deposition varied with Reynolds number, probably due to changes in the flow field with Reynolds number. For these reasons, an empirical Reynolds number correction was employed to the collected data where Stokes number is multiplied by Reynolds number, Re. to the power of 0.37 That is to say:

$$R_e \ = \ \rho \ U_{mean} \ D_{mean} \ / \ \mu \qquad\qquad (6)$$

Formula (6) may also be expressed as:

$$R_e \ = \ (2\rho Q \ / \ \mu)(L \ / \ V)^{0.5} \qquad\qquad (6a)$$

And the Stokes number is therefore given as:

$$Stk = (\rho \ d_p{}^2 Q \ / \ 36\mu)(\pi L^3 \ / \ V^3)^{0.5} \qquad\qquad (4a)$$

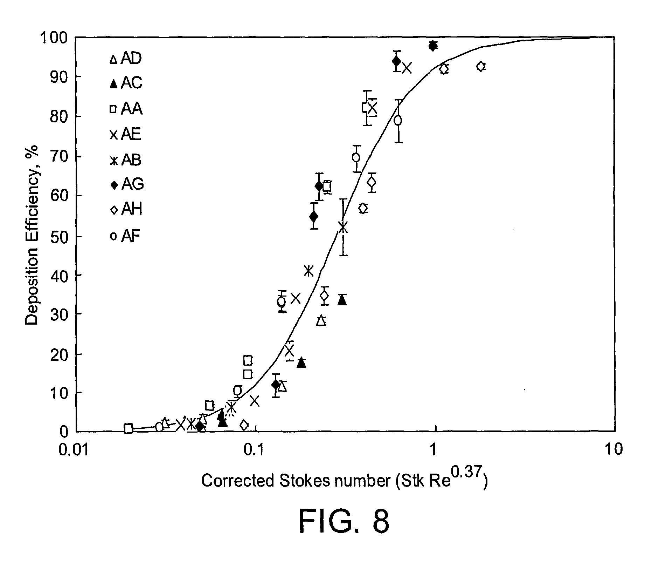

The resulting curves are shown in Figure 8 together with the least square curve fit function $\eta = 100-100/(11.5 (Stk \ Re^{0.37})^{1.912}+1)$, where $\eta$ is deposition efficiency. The good collapse of all data onto nearly a single curve indicates that by knowing a subject's mouth and throat mean equivalent diameter $D_{mean}$ as well as particle size and inhalation flow rate ($U_{mean}$), it is possible to more accurately predict throat deposition. The use of equations (1), (6), (6a) and (4a) to predict mouth-throat deposition requires knowing the volume V and centreline path-length L of the given mouth-throat. When this information is not available, average values of these parameters may be used in an approximation of the method.

2. Second reference database

In accord with the method herein, a second reference dataset comprising throat physical parameter data was collected from the throats of a sample of six subjects. The general steps involved in assembling this second reference dataset correspond closely to those used in compiling the first reference dataset and may be better understood by reference to the flow diagram of Figure 3.

The sample group of subjects was selected 220 to comprise subjects of different builds (i.e. large and small adults) to give a good range of throat sizes. Physical parameter data was then collected 222 for each subject by use of Magnetic Resonance Imaging (MRI) of the throat airway of each subject in the sample group. MRI Imaging is a known technique for collection of such data. The physical parameter data collected for each subject comprised: the volume of the airway defined by the subject throat; and the average cross-sectional area of that airway measured in the plane perpendicular to the airway wall. The physical parameter data for each subject was stored as a second dataset on a computerized database 224 and used to define six throat models designated Throats A to F.

For the reason that the particle size distribution of the emitted dose from inhaled dry powder inhalers are formulation and device specific, throat deposition data was also collected relevant to each subject 226 of the same initial sample by requiring a defined particle sample to be inhaled through each of the model Throats A to F defined by and constructed according to the previously collected physical throat parameters of each subject under measurable inhalation flow conditions. In more detail, both the total dose emitted from the inhaler and ex-throat (beyond the throat) dose was obtained for each of Throats A to F. The so-obtained throat deposition data for each subject throat was stored as a corresponding dataset on the computerized database 228.

A second reference computerized database was thus, assembled to comprise throat physical parameter and corresponding throat deposition data for each subject in the sample group. Using appropriate plots, the throat deposition data was plotted against the throat physical parameter data to give reference curves 230 that might be used in a predictive sense (e.g. to predict throat deposition for a new patient for whom suitable throat physical parameter data is known).

A summary of the relevant data (obtained for a particular inhalation device and formulation) for each of Throats A to F is shown in Table 1 below:

Table 1

| Throat | Throat volume (cm3) | Velocity within throat (cms-1) | Total emitted dose (% label) | Ex-throat dose (% label) |
|--------|---------------------|-------------------------------|------------------------------|---------------------------|
| A | 49.4 | 623.6 | 93.2 | 13 |
| B | 64.9 | 769.2 | 90.7 | 9.6 |
| C | 106.8 | 417.6 | 91.3 | 22.6 |
| D | 47.5 | 838.1 | 87.7 | 15.3 |
| E | 106.8 | 417.6 | 85.3 | 21.3 |
| F | 33.7 | 1303.7 | 89.7 | 5.1 |

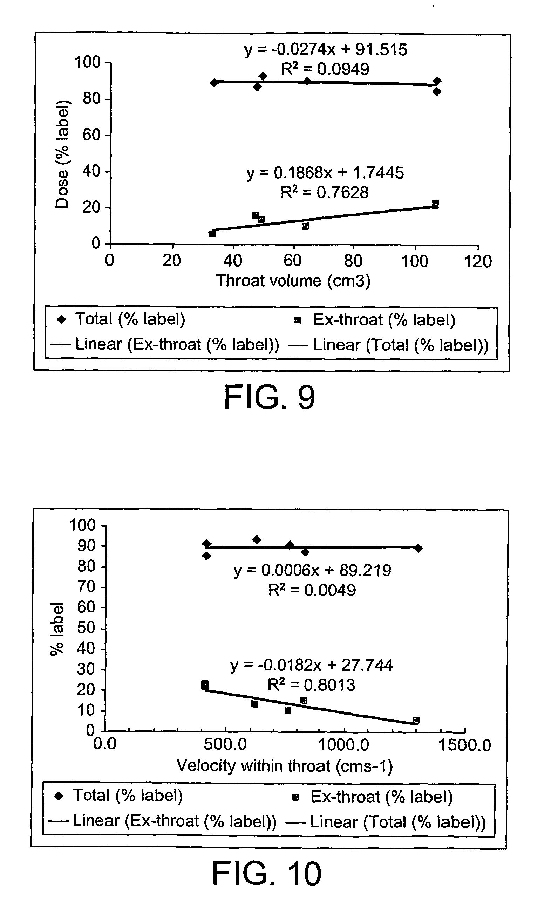

The data of Table 1 was used to construct reference curves shown respectively at Figures 9 and 10, wherein Figure 9 employs the throat volume data and Figure 10 employs the velocity within throat data of Table 1. Good linear correlation is shown on both curves.

Acoustic imaging

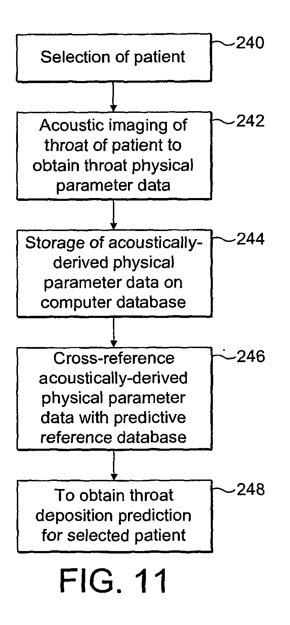

In accord with the method herein, a further (i.e. new) patient was then selected for whom it was desired to predict the tendency of the defined particle sample to deposit within their throat by use of acoustic imaging measurements for this patient and reference to the earlier created database. The steps involved in making this predictive assessment may be better understood by reference to the flow diagram of Figure 11.

The airway defined by the throat of the selected patient 240 was then mapped using acoustic reflection imaging 242 such that physical parameter data was obtained corresponding to the volume of the airway defined by the patient throat; the cross-section area of that airway measured in plane perpendicular to the airway wall; and length of that airway also measured in the central sagittal plane. The acoustically-derived physical parameter data for each patient was stored as a dataset on a computerized database 244.

In more detail, the acoustic reflection imaging technique comprised use of the Acoustic Pharyngometry apparatus sold by Hood Laboratories of 575 Washington St, Pembroke, MA 02359, United States of America under the trade name Eccovision. The apparatus was used in accord with the Operating Manual provided therewith, which involves taking acoustic measurements of throat characteristics during oral inhalation by the patient. The apparatus is further described in PCT Patent Application No. WO 94/09700 also in the name of Hood Laboratories (incorporated herein by reference).

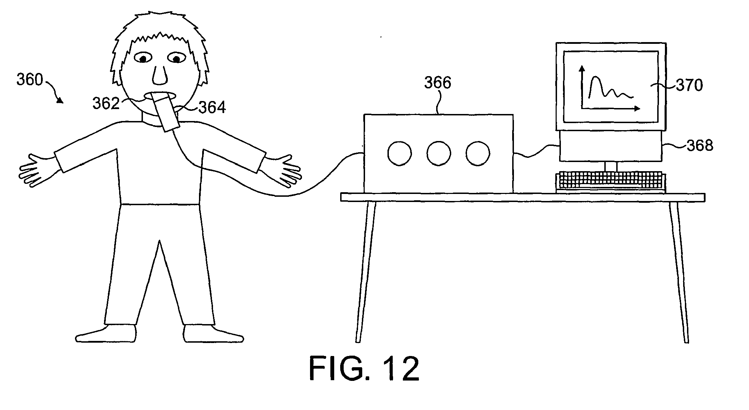

A schematic representation of the use of the acoustic imaging apparatus is shown at Figure 12. Patient 360 exhales into disposable acoustic pipe 362 and such exhaled breath is received by the acoustic chamber 364 of the apparatus, which includes electronic components comprising launching transducer; acoustic pressure wave sensing transducer; and signal processor (details of electronic components not visible). The acoustic chamber 364 connects via wiring to control box 366, which in turn connects to personal computer 368 and monitor 370. The acoustically-generated patient throat data is then stored and analysed by means of the personal computer 368 and displayed on the monitor 370 either pictorially, graphically or as a list of data.

Predictive matching

The physical parameter data defining the airway of the new patient is then cross-referenced with the data of a reference dataset 246. By matching of the new patient physical parameter data with the data of the reference dataset (e.g. by fitting to appropriate reference curves such as those shown at Figures 5 to 8; or Figures 9 and 10) the tendency for particle deposition to occur at the throat of the new patient is predicted 248.

By way of a first example, Table 2 shows the results of the predicted ex-throat dose for a set of six patients (P1 to P6) for whom velocity within the throat data was collected by use of acoustic pharyngometry (as described above) and for whom reference matching was made with the second reference dataset of Table 1 and Figure 10.

Table 2

| Patient | Velocity within throat (cms-1) | Predicted ex-throat dose (% label) |
|---------|-------------------------------|-------------------------------------|
| P1 | 311.0 | 22.1 |

(continued)

| Patient | Velocity within throat (cms-1) | Predicted ex-throat dose (% label) |
|---------|-------------------------------|-----------------------------------|
| P2 | 472.3 | 19.1 |
| P3 | 679.9 | 15.4 |
| P4 | 1134.5 | 7.1 |
| P5 | 396.4 | 20.5 |
| P6 | 493.0 | 18.8 |

By way of a second example, Table 3 shows the results of the predicted ex-throat dose for a set of six patients (P1 to P6) for whom throat volume data was collected by use of acoustic pharyngometry (as described above) and for whom reference matching was made with the second reference dataset of Table 1 and Figure 9.

Table 3

| Patient | Total throat volume (cm3) | Predicted ex-throat dose (% label) |
|---------|---------------------------|-----------------------------------|
| P1 | 102.9 | 21.0 |
| P2 | 114.0 | 23.0 |
| P3 | 66.1 | 14.1 |
| P4 | 39.3 | 9.1 |
| P5 | 88.5 | 18.3 |
| P6 | 76.6 | 16.1 |

Good correspondence is noted for the predictions of ex-throat dose for each patient throat (P1 to P6) based respectively upon velocity within throat (Table 2) and total throat volume (Table 3).

Comparison of MRI and acoustically derived throat parameter data

A further sample of seven patients (T1 to T7) was selected and throat parameter data relating to total throat volume and average throat diameter was obtained using (1) MRI measurements; and (2) acoustic measurements (both measured using the particular MRI and acoustic methods described in detail hereinbefore).
The so-derived throat physical parameter is reproduced at Table 4. below. Good correspondence is shown between the data obtained using the two different methods of obtaining the throat physical parameter data.

Table 4

| Throat ID | (1) MRI | | (2) Acoustic | | Difference in total volume | Difference in average diameter |
|-----------|---------|---|--------------|---|--------------|-------------|
| | Total volume (cm3) | Average diameter (cm) | Total volume (cm3) | Average diameter (cm) | | |
| T1 | 31.1 | 1.5 | 31.3 | 1.7 | 0.5% | 16.6% |
| T2 | 49.2 | 1.7 | 49.4 | 1.9 | 0.4% | 14.3% |
| T3 | 33.8 | 1.4 | 33.7 | 1.5 | -0.1% | 8.1% |
| T4 | 34.9 | 1.5 | 35.2 | 1.6 | 0.8% | 8.4% |
| T5 | 44.9 | 1.6 | 45.4 | 1.8 | 1.1% | 12.3% |
| T6 | 47.8 | 1.7 | 47.5 | 1.9 | -0.7% | 10.8% |
| T7 | 23.4 | 1.3 | 24.8 | 1.4 | 6.2% | 8.8% |

Uses of the method herein

The method of the present invention is suitable for use in the assessment of inhalation-type medicament dispenser devices, such as those suitable for the delivery of medicament for the treatment of respiratory disorders such as asthma and chronic obstructive pulmonary disease (COPD), bronchitis and chest infections and for the systemic treatment of insulin-dependent diabetes.

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6$\alpha$, 9$\alpha$-difluoro-11$\beta$-hydroxy-16$\alpha$-methyl-3-oxo-17$\alpha$-propionyloxy-androsta-1,4-diene-17$\beta$-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g. 2R,3R,4S,5R)-2-[6-Amino-2-(1 S-hydroxymethyl-2-phenyl-ethylamino)-purin-9-yl]-5-(2-ethyl-2H-tetrazol-5-yl)-tetrahydro-furan-3,4-diol (e.g. as maleate); $\alpha_4$ integrin inhibitors e.g. (2S)-3-[4-({[4-(aminocarbonyl)-1-piperidinyl]carbonyl}oxy)phenyl]-2-[((2S)-4-methyl-2-{2-(2-methylphenoxy) acetyl]amino} pentanoyl)amino] propanoic acid (e.g. as free acid or potassium salt), diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Suitable medicament components for the treatment of respiratory disorders are particularly selected from the group consisting of anti-inflammatory agents (for example a corticosteroid or an NSAID), anticholinergic agents (for example, an $M_1$, $M_2$, $M_1/M_2$ or $M_3$ receptor antagonist), other $\beta_2$-adrenoreceptor agonists, antiinfective agents (e.g. an antibiotic or an antiviral), antihistamines, and mixtures thereof.

Suitable anti-inflammatory agents include corticosteroids and NSAIDs. Suitable corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, 6$\alpha$,9$\alpha$-difluoro-17$\alpha$-[(2-furanylcarbonyl)oxy]-11$\beta$-hydroxy-16$\alpha$-methyl-3-oxo-androsta-1,4-diene-17$\beta$-carbothioic acid S-fluoromethyl ester, 6$\alpha$,9$\alpha$-difluoro-11$\beta$-hydroxy-16$\alpha$-methyl-3-oxo-17$\alpha$-propionyloxy-androsta-1,4-diene-17$\beta$-carbothioic acid S-(2-oxo-tetrahydro-furan-3S-yl) ester, beclomethasone esters (e.g. the 17-propionate ester or the 17,21-dipropionate ester), budesonide, flunisolide, mometasone esters (e.g. the furoate ester), triamcinolone acetonide, rofleponide, ciclesonide, butixocort propionate, RPR-106541, and ST-126. Preferred corticosteroids include fluticasone propionate, 6$\alpha$,9$\alpha$-difluoro-11$\beta$-hydroxy-16$\alpha$-methyl-17$\alpha$-[(4-methyl-1,3-thiazole-5-carbonyl)oxy]-3-oxo-androsta-1,4-diene-17$\beta$-carbothioic acid S-fluoromethyl ester and 6$\alpha$,9$\alpha$-difluoro-17$\alpha$-[(2-furanylcarbonyl)oxy]-11$\beta$-hydroxy-16$\alpha$-methyl-3-oxo-androsta-1,4-diene-17$\beta$-carbothioic acid S-fluoromethyl ester, more preferably 6$\alpha$,9$\alpha$-difluoro-17$\alpha$-[(2-furanylcarbonyl)oxy]-11$\beta$-hydroxy-16$\alpha$-methyl-3-oxo-androsta-1,4-diene-17$\beta$-carbothioic acid S-fluoromethyl ester.

Suitable NSAIDs include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis, iNOS inhibitors, tryptase and elastase inhibitors, beta-2 integrin antagonists and adenosine receptor agonists or antagonists (e.g. adenosine 2a agonists), cytokine antagonists (e.g. chemokine antagonists) or inhibitors of cytokine synthesis. Suitable other $\beta_2$-adrenoreceptor agonists include salmeterol (e.g. as the xinafoate), salbutamol (e.g. as the sulphate or the free base), formoterol (e.g. as the fumarate), fenoterol or terbutaline and salts thereof.

Suitable PDE4-specific inhibitors include any compound that is known to inhibit the PDE4 enzyme or which is discovered to act as a PDE4 inhibitor, and which are only PDE4 inhibitors, not compounds which inhibit other members of the PDE family as well as PDE4. Generally it is preferred to use a PDE4 inhibitor which has an $IC_{50}$ ratio of about 0.1 or greater as regards the $IC_{50}$ for the PDE4 catalytic form which binds rolipram with a high affinity divided by the $IC_{50}$ for the form which binds rolipram with a low affinity. For the purposes of this disclosure, the cAMP catalytic site which binds R and S rolipram with a low affinity is denominated the "low affinity" binding site (LPDE 4) and the other form of this catalytic site which binds rolipram with a high affinity is denominated the "high affinity" binding site (HPDE 4). This term "HPDE4" should not be confused with the term "hPDE4" which is used to denote human PDE4. A method for

determining IC$_{50}$s ratios is set out in US patent 5,998,428 which is incorporated herein in full by reference as though set out herein. See also PCT application WO 00/51599 for an another description of said assay.

Suitable PDE4 inhibitors include those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an IC$_{50}$ ratio of about 0.1 or greater as regards the IC$_{50}$ for the PDE4 catalytic form which binds rolipram with a high affinity divided by the IC$_{50}$ for the form which binds rolipram with a low affinity.

A further refinement of this standard is that of one wherein the PDE4 inhibitor has an IC$_{50}$ ratio of about 0.1 or greater; said ratio is the ratio of the IC$_{50}$ value for competing with the binding of 1nM of [$^3$H]R-rolipram to a form of PDE4 which binds rolipram with a high affinity over the IC$_{50}$ value for inhibiting the PDE4 catalytic activity of a form which binds rolipram with a low affinity using 1 $\mu$M[$^3$H]-cAMP as the substrate.

Most suitable PDE4 inhibitors are those which have an IC$_{50}$ ratio of greater than 0.5, and particularly those compounds having a ratio of greater than 1.0. Preferred compounds are *cis* 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]; these are examples of compounds which bind preferentially to the low affinity binding site and which have an IC$_{50}$ ratio of 0.1 or greater.

Other suitable medicament compounds include: cis-4-cyano-4-[3-(cyclopentyloxy)-4-methoxyphenyl]cyclohexane-1-carboxylic acid (also known as cilomalast) disclosed in U.S. patent 5,552,438and its salts, esters, pro-drugs or physical forms; AWD-12-281 from elbion (Hofgen, N. et al. 15th EFMC Int Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505, the disclosure of which is hereby incorporated by reference) from Byk-Gulden; Pumafentrine, (-)-p-[(4$a$R*,10$b$S*)-9-ethoxy-1,2,3,4,4a, 10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther,1998, 284(1): 162), and T2585.

Suitable anticholinergic agents are those compounds that act as antagonists at the muscarinic receptor, in particular those compounds, which are antagonists of the M$_1$ and M$_2$ receptors. Exemplary compounds include the alkaloids of the belladonna plants as illustrated by the likes of atropine, scopolamine, homatropine, hyoscyamine; these compounds are normally administered as a salt, being tertiary amines.

Particularly suitable anticholinergics include ipratropium (e.g. as the bromide), sold under the name Atrovent, oxitropium (e.g. as the bromide) and tiotropium (e.g. as the bromide) (CAS-139404-48-1). Also of interest are: methantheline (CAS-53-46-3), propantheline bromide (CAS- 50-34-9), anisotropine methyl bromide or Valpin 50 (CAS-80-50-2), clidinium bromide (Quarzan, CAS-3485-62-9), copyrrolate (Robinul), isopropamide iodide (CAS-71-81-8), mepenzolate bromide (U.S. patent 2,918,408), tridihexethyl chloride (Pathilone, CAS-4310-35-4), and hexocyclium methylsulfate (Tral, CAS-115-63-9). See also cyclopentolate hydrochloride (CAS-5870-29-1), tropicamide (CAS-1508-75-4), trihexyphenidyl hydrochloride (CAS-144-11-6), pirenzepine (CAS-29868-97-1), telenzepine (CAS-80880-90-9), AF-DX 116, or methoctramine, and the compounds disclosed in WO01/04118.

Suitable antihistamines (also referred to as H$_1$-receptor antagonists) include any one or more of the numerous antagonists known which inhibit H$_1$-receptors, and are safe for human use. All are reversible, competitive inhibitors of the interaction of histamine with H$_1$-receptors. Examples include ethanolamines, ethylenediamines, and alkylamines. In addition, other first generation antihistamines include those which can be characterized as based on piperizine and phenothiazines. Second generation antagonists, which are non-sedating, have a similar structure-activity relationship in that they retain the core ethylene group (the alkylamines) or mimic the tertiary amine group with piperizine or piperidine. Exemplary antagonists are as follows:

Ethanolamines: carbinoxamine maleate, clemastine fumarate, diphenylhydramine hydrochloride, and dimenhydrinate.

Ethylenediamines: pyrilamine amleate, tripelennamine HCl, and tripelennamine citrate.

Alkylamines: chlropheniramine and its salts such as the maleate salt, and acrivastine.

Piperazines: hydroxyzine HCl, hydroxyzine pamoate, cyclizine HCl, cyclizine lactate, meclizine HCl, and cetirizine HCl.

Piperidines: Astemizole, levocabastine HCl, loratadine or its descarboethoxy analogue, and terfenadine and fexofenadine hydrochloride or another pharmaceutically acceptable salt.

Azelastine hydrochloride is yet another $H_1$ receptor antagonist which may be used in combination with a PDE4 inhibitor.

Particularly suitable anti-histamines include methapyrilene and loratadine.

Generally, medicament particles suitable for delivery to the bronchial or alveolar region of the lung have an aerodynamic diameter of less than 10 micrometers, preferably less than 6 micrometers. Other sized particles may be used if delivery to other portions of the respiratory tract is also desired, such as the nasal cavity, mouth or throat. The medicament may be delivered as pure drug, but suitably where in dry powder form medicaments are delivered together with excipients (carriers) which are suitable for inhalation. Suitable excipients include organic excipients such as polysaccharides (i.e. starch, cellulose and the like), lactose, glucose, mannitol, amino acids, and maltodextrins, and inorganic excipients such as calcium carbonate or sodium chloride. Lactose is a preferred excipient.

Particles of powdered medicament and/or excipient may be produced by conventional techniques, for example by micronisation, milling or sieving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well-known methods, such as by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 100:1 to 1:1 may be used. At very low ratios of excipient to drug, however, the drug dose reproducibility may become more variable.

Inhaler devices herein are particularly suitable for dispensing medicament for the treatment of respiratory disorders such as disorders of the lungs and bronchial tracts including asthma and chronic obstructive pulmonary disorder (COPD). In another aspect, the inhaler devices are suitable for dispensing medicament for the treatment of a condition requiring treatment by the systemic circulation of medicament, for example migraine, diabetes, pain relief e.g. inhaled morphine.

The amount of any particular medicament compound or a pharmaceutically acceptable salt, solvate or physiologically functional derivative thereof which is required to achieve a therapeutic effect will, of course, vary with the particular compound, the route of administration, the subject under treatment, and the particular disorder or disease being treated. The medicaments for treatment of respiratory disorders herein may for example, be administered to a human patient by inhalation at a dose of from 0.0005mg to 10 mg, preferably 0.005mg to 0.5mg. The dose range for adult humans is generally from 0.0005 mg to 100mg per day and preferably 0.01 mg to 1 mg per day.

It will be understood that the present disclosure is for the purpose of illustration only and the invention extends to modifications, variations and improvements thereto within the scope of the appended claims.

**Claims**

1. A method for predicting the tendency of inhaled particles to deposit within a first patient's throat when said particles are inhaled through an airway defined by said first patient's throat, said method comprising
determining at least one internal physical parameter of said airway defined by the first patient's throat by means of acoustic imaging of the airway defined by the first patient's throat; and
matching said at least one internal physical parameter of the airway of the first patient's throat with a dataset comprising pre-determined data relating to the corresponding internal physical parameter for the throat of at least one other patient,
wherein said dataset also comprises pre-determined data relating to the tendency of said inhaled particles to deposit within said at least one other patient's throat, and said matching thereby enables prediction of the tendency for the inhaled particles to deposit within the first patient's throat.

2. A method according to claim 1, wherein said acoustic imaging comprises acoustic reflection imaging.

3. A method according to claim 1 or 2, wherein the acoustic imaging is by by means of acoustic pharyngometry.

4. A method according to any of claims 1 to 3, wherein the at least one internal physical parameter is selected from the group consisting of throat volume, throat cross-sectional area and throat length.

5. A method according to any of claims 1 to 4, wherein the dataset comprises said pre-determined data relating to the corresponding internal physical parameter for the throat of at least ten other patients.

6. A method according to any of claims 1 to 5, wherein the pre-determined data relating to the corresponding internal physical parameter is collected by use of Magnetic Resonance Imaging (MRI) of the throat airway of the at least one other patient.

7. A method according to any of claims 1 to 6, wherein said data relating to the tendency of said inhaled particles to deposit within the at least one other patient's throat is obtained by use of a laboratory model reconstruction thereof.

8. A method according to any of claims 1 to 7, wherein said matching is by use of a curve-fitting method.

9. A method according to any of claims 1 to 8,
   wherein the dataset comprises data relevant to each of plural patients and is assembled by

   (i) determining at least one internal physical parameter of the airway defined by the throat of each other patient; and
   (ii) determining the tendency of inhaled particles to deposit within the throat of each other patient.

10. A method according to any of claims 1 to 9, wherein the dataset comprising physical parameter and deposition of inhaled particles data relevant to the at least one other patient is obtained by
    measuring the volume (V) of the airway defined by the throat of the at least one other patient;
    measuring the path length (L) of a central line of the throat airway in the mid-sagittal plane;
    measuring the flow rate (Q) of said particles or the airflow in which said particles are suspended;
    calculating a mean throat diameter ($D_{mean}$) by means of the formula

$$D_{mean} = 2 (V / \pi L)^{0.5} \qquad\qquad (1)$$

calculating a mean particle flow velocity ($U_{mean}$) by means of the formula;

$$U_{mean} = QL / V \qquad\qquad (2)$$

and predicting the amount of particle deposition (P) at the throat by correlating terms defined by the formula

$$P = f(U_{mean} / D_{mean}) \qquad\qquad (3)$$

wherein P is a function of $U_{mean}$ and $D_{mean}$.

**Patentansprüche**

1. Verfahren zur Vorhersage der Tendenz von inhalierten Partikeln, sich im Rachen eines ersten Patienten abzusetzen, wenn die Partikel durch einen durch den Rachen des ersten Patienten definierten Luftweg inhaliert werden, wobei das Verfahren folgendes umfasst:

   Bestimmen wenigstens eines internen physischen Parameters des durch den Rachen des ersten Patienten definierten Luftwegs mittels akustischer Bildgebung des durch den Rachen des ersten Patienten definierten Luftwegs;
   Abgleichen des wenigstens einen internen physischen Parameters des Luftwegs des Rachens des ersten Patienten mit einem Datensatz, der vorbestimmte Daten umfasst, die den entsprechenden internen physischen Parameter des Rachens wenigstens eines anderen Patienten betreffen,

wobei der Datensatz ebenfalls vorbestimmte Daten umfasst, die die Tendenz der inhalierten Partikel, sich im Rachen des wenigstens einen anderen Patienten abzusetzen, betreffen, und der Abgleich **dadurch** die Vorhersage der Tendenz für die inhalierten Partikel, sich innerhalb des Rachens des ersten Patienten abzusetzen, möglich macht.

2. Verfahren gemäss Anspruch 1, wobei die akustische Bildgebung eine akustische Reflexionsbildgebung umfasst.

3. Verfahren gemäss Anspruch 1 oder 2, wobei die akustische Bildgebung mittels akustischer Pharyngometrie erfolgt.

4. Verfahren gemäss einem der Ansprüche 1 bis 3, wobei der wenigstens eine interne physische Parameter ausgewählt ist aus der Gruppe, bestehend aus Rachenvolumen, Querschnittsfläche des Rachens und Rachenlänge.

5. Verfahren gemäss einem der Ansprüche 1 bis 4, wobei der Datensatz die vorbestimmten Daten umfasst, die den entsprechenden internen physischen Parameter des Rachens von wenigstens 10 anderen Patienten betreffen.

6. Verfahren gemäss einem der Ansprüche 1 bis 5, wobei die vorbestimmten Daten, die den entsprechenden internen physischen Parameter betreffen, durch kernspintomographische Aufnahme (MRI) des Rachenluftwegs des wenigstens einen anderen Patienten gewonnen werden.

7. Verfahren gemäss einem der Ansprüche 1 bis 6, wobei die Daten, die die Tendenz der inhalierten Partikel, sich im Rachen des wenigstens einen anderen Patienten abzusetzen, betreffen, durch Verwendung einer Labormodellrekonstruktion davon erhalten werden.

8. Verfahren gemäss einem der Ansprüche 1 bis 7, wobei die Abgleichung durch Verwendung eines Verfahrens zur Kurvenanpassung erfolgt.

9. Verfahren gemäss einem der Ansprüche 1 bis 8, wobei der Datensatz Daten umfasst, die für jeden der Vielzahl von Patienten von Relevanz sind, und zusammengestellt ist durch

(i) Bestimmen wenigstens eines internen physischen Parameters des durch den Rachen jedes anderen Patienten definierten Luftwegs; und
(ii) Bestimmen der Tendenz der inhalierten Partikel, sich im Rachen jedes anderen Patienten abzusetzen.

10. Verfahren gemäss einem der Ansprüche 1 bis 9, wobei der Datensatz, der die für den wenigstens einen anderen Patienten relevanten Daten hinsichtlich des physischen Parameters und der Ablagerung der inhalierten Partikel umfasst, erhalten wird durch
Messen des Volumens (V) des durch den Rachen des wenigstens einen anderen Patienten definierten Luftwegs;
Messen der Weglänge (L) einer zentralen Linie des Rachenluftwegs in der Mittsagittalebene;
Messen der Fliessrate (Q) der Partikel oder des Luftstroms, in dem die Partikel suspendiert sind;
Berechnen eines mittleren Rachendurchmessers ($D_{mittel}$) anhand der Formel:

$$D_{mittel} = 2\ (V/\pi L)^{0,5} \qquad (1)$$

Berechnen einer mittleren Partikelfliessgeschwindigkeit ($U_{mittel}$) anhand der Formel:

$$U_{mittel} = QL/V \qquad (2)$$

und Vorhersagen der Menge an Partikelablagerung (P) im Rachen durch Korrelieren von Termen, die durch die Formel

$$P = f(U_{mittel}/D_{mittel}) \quad (3)$$

definiert sind, wobei P eine Funktion von $U_{mittel}$ und $D_{mittel}$ ist.

## Revendications

**1.** Procédé de prédiction de la tendance des particules inhalées à se déposer dans la gorge d'un premier patient quand lesdites particules sont inhalées à travers un conduit aérien défini par ladite gorge du premier patient, ledit procédé comprenant :

la détermination d'au moins un paramètre physique interne dudit conduit aérien, défini par la gorge du premier patient, au moyen d'une imagerie acoustique du conduit aérien, défini par la gorge du premier patient ; et
l'adaptation dudit au moins un paramètre physique interne du conduit aérien de la gorge du premier patient à un ensemble de données comprenant des données prédéterminées relatives au paramètre physique interne correspondant pour la gorge d'au moins un autre patient,
dans lequel ledit ensemble de données comprend également des données prédéterminées concernant la tendance desdites particules inhalées à se déposer dans la gorge d'au moins un autre patient, et ladite adaptation permettant ainsi la prédiction de la tendance des particules inhalées à se déposer dans la gorge du premier patient.

**2.** Procédé selon la revendication 1, dans lequel ladite imagerie acoustique comprend une imagerie par réflexion acoustique.

**3.** Procédé selon la revendication 1 ou 2, dans lequel l'imagerie acoustique s'effectue au moyen d'une pharyngométrie acoustique.

**4.** Procédé selon l'une quelconque des revendications 1 à 3, dans lequel ledit au moins un paramètre physique interne est choisi dans le groupe constitué du volume de la gorge, la superficie en coupe transversale de la gorge et la longueur de la gorge.

**5.** Procédé selon l'une quelconque des revendications 1 à 4, dans lequel l'ensemble de données comprend lesdites données prédéterminées relatives au paramètre physique interne correspondant pour la gorge d'au moins dix autres patients.

**6.** Procédé selon l'une quelconque des revendications 1 à 5, dans lequel les données prédéterminées concernant le paramètre physique interne correspondant sont collectées en utilisant l'imagerie par résonance magnétique (IRM) du conduit aérien de la gorge dudit au moins un autre patient.

**7.** Procédé selon l'une quelconque des revendications 1 à 6, dans lequel lesdites données concernant la tendance desdites particules inhalées à se déposer dans ladite gorge d'au moins un autre patient est obtenue en utilisant une reconstruction par modèle de laboratoire de celle-ci.

**8.** Procédé selon l'une quelconque des revendications 1 à 7, dans lequel ladite adaptation s'effectue en utilisant un procédé d'ajustement analytique.

**9.** Procédé selon l'une quelconque des revendications 1 à 8, dans lequel l'ensemble de données comprend des données pertinentes à chacun des différents patients et est assemblé en

(i) déterminant au moins un paramètre physique interne du conduit aérien défini par la gorge de chaque autre patient ; et
(ii) déterminant la tendance des particules inhalées à se déposer dans la gorge de chaque patient.

**10.** Procédé selon l'une quelconque des revendications 1 à 9, dans lequel l'ensemble de données, comprenant un paramètre physique et les données concernant le dépôt de particules inhalées concernant ledit au moins un autre patient est obtenu en

mesurant le volume (V) du conduit aérien défini par la gorge dudit au moins un autre patient ;

mesurant la longueur de parcours (L) d'une ligne centrale du conduit aérien de la gorge dans le plan demi-sagittal ;

mesurant le débit (Q) desdites particules ou le flux d'air dans lequel lesdites particules sont suspendues ;

calculant un diamètre moyen de gorge ($D_{moyen}$) à l'aide de la formule

$$D_{moyen} = 2 \ (V \ / \ \pi L)^{0,5} \qquad\qquad (1)$$

calculant une vitesse de flux de particules moyenne ($U_{moyenne}$) à l'aide de la formule :

$$U_{moyenne} \ = \ QL/V \qquad\qquad (2)$$

et en prédisant la quantité de dépôt de particules (P) dans la gorge par des termes de corrélation définis par la formule

$$P \ = \ f(U_{moyenne} \ / \ D_{moyen}) \qquad\qquad (3)$$

où P est une fonction de $U_{moyenne}$ et de $D_{moyen}$.

FIG. 1

FIG. 2a    FIG. 2b    FIG. 2c

EP 1 696 988 B1

Selection of patient sample group ~220

Collection of throat physical parameter data for each patient by MRI imaging ~222

Storage of physical parameter data on computer database ~224

Collection of throat deposition data for each patient by use of model airway ~226

Storage of throat deposition data on computer database ~228

Plotting of physical parameter data versus throat deposition data to give predictive reference curves ~230

# FIG. 3

FIG. 4

Deposition Efficiency, %

Inertial Parameter, $pd_p^2Q$ (g μm$^2$s$^{-1}$)

- ■ AA
- ✳ AB
- ▲ AC
- △ AD
- ✕ AE
- ○ AF
- ◆ AG
- ◇ AH

FIG. 5

FIG. 6

EP 1 696 988 B1

FIG. 7

FIG. 8

EP 1 696 988 B1

$$y = -0.0274x + 91.515$$
$$R^2 = 0.0949$$

$$y = 0.1868x + 1.7445$$
$$R^2 = 0.7628$$

FIG. 9

$$y = 0.0006x + 89.219$$
$$R^2 = 0.0049$$

$$y = -0.0182x + 27.744$$
$$R^2 = 0.8013$$

FIG. 10

Selection of patient ⌐~240

↓

Acoustic imaging of throat of patient to obtain throat physical parameter data ⌐~242

↓

Storage of acoustically-derived physical parameter data on computer database ⌐~244

↓

Cross-reference acoustically-derived physical parameter data with predictive reference database ⌐~246

↓

To obtain throat deposition prediction for selected patient ⌐~248

# FIG. 11

FIG. 12

## REFERENCES CITED IN THE DESCRIPTION

### Patent documents cited in the description

- WO 0174247 A
- WO 9409700 A
- US 5998428 A
- WO 0051599 A
- US 5552438 A
- WO 9916766 A
- WO 9947505 A
- US 2918408 A
- WO 0104118 A

### Non-patent literature cited in the description

- **Brooks LJ ; Castile RG ; Glass GM ; Griscom NT ; Wohl ME ; Fredberg JJ.** Reproducibility and accuracy of airway area by acoustic reflection. *J Appl Physiol.,* September 1984, vol. 57 (3), 777-87
- **D'Urzo AD ; Lawson VG ; Vassal KP ; Rebuck AS ; Slutsky AS ; Hoffstein V.** Airway area by acoustic response measurements and computerized tomography. *Am Rev Respir Dis.,* vol. 135 (2), 392-5
- **Hoffstein V ; Fredberg JJ.** The acoustic reflection technique for non-invasive assessment of upper airway area. *Eur Respir J.,* vol. 4 (5), 602-11
- **Zhou Y ; Daubenspeck JA.** Measurement of upper airway movement by acoustic reflection. *Ann Biomed Eng.,* vol. 23 (1), 85-94
- **Louis B ; Glass GM ; Fredberg JJ.** Pulmonary airway area by the two-microphone acoustic reflection method. *J Appl Physiol.,* vol. 76 (5), 2234-40
- **Marshall I ; Maran NJ ; Martin S ; Jan MA ; Rimmington JE ; Best JJ ; Drummond GB ; Douglas NJ.** Acoustic reflectometry for airway measurements in man: implementation and validation. *Physiol Meas.,* vol. 14 (2), 157-69
- *J Appl Physiol,* vol. 88 (4), 1457-66
- **Ehtezazi T ; Horsfield MA ; Barry P ; O Callaghan CO.** *Proceedings of Drug Delivery to the lungs XI,* 2000, 90-93
- **de Lange EE ; Mugler JP III ; Brookeman JR ; Knight-Scott J ; Truwit JD ; Teates CD ; Daniel TM ; Bogorad PL ; Cates GD.** *Radiology,* 1999, vol. 210 (3), 851-857
- **McRobbie DW ; Pritchard S ; Quest R.** Studies of the human oropharyngeal airspaces using magnetic resonance imaging I. Validation of a three-dimensional MRI method for producing ex vivo virtual and physical casts of the oropharyngeal airways during inspiration. *J Aerosol Medicine,* 2003, vol. 16, 399-413
- **McRobbie DW ; Pritchard SE ; Quest RA.** Volumetric studies of the oropharyngeal airways by 3D MRI. *International Society for Aerosols in Medicine, 13th International Congress,* 17 September 2001
- **McRobbie D.W. ; Quest R.A. ; Pritchard S.** Pulse Sequences for Respiratory Gated MR Virtual Bronchoscopy. *International Society for Megnetic Resonance in Medicine,* 2000
- **B Grgic ; W H Finlay ; P K P Burnell ; AF Heenan.** In Vitro Intersubject and Intrasubject Deposition Measurements in Realistic Mouth-Throat Geometries. *Aerosol Science,* 2004, vol. 35, 1025-1040
- **Stapleton KW ; Guentsch E ; Hoskinson M.K. ; Finlay WH.** On the Suitability of K-E Turbulence Modelling for Aerosol Dispersion on the Mouth and Throat: A Comparison with Experiment. *J. Aerosol Sci,* 2000, vol. 31, 739-749
- **Hofgen, N. et al.** *15th EFMC Int Symp Med Chem,* 06 September 1998, 98
- **Landells, L.J. et al.** *Eur Resp J [Annu Cong Eur Resp Soc,* 19 September 1998, vol. 12 (28
- **Fuji, K. et al.** *J Pharmacol Exp Ther,* 1998, vol. 284 (1), 162